# EUROPEAN PATENT SPECIFICATION

(11) **EP 1 904 031 B1**
(45) Date of publication and mention of the grant of the patent: **08.01.2014**
(21) Application number: 06755153.1
(22) Date of filing: 11.05.2006
(51) Int. Cl.: A61K 9/12, A61K 9/70, A61K 47/14, A61K 47/28

(54) **A SUBSTANTIALLY WATER-FREE PRESSURISED PROPELLANT MIXTURE**
IM WESENTLICHEN WASSERFREIES DRUCKBEAUFSCHLAGTES TREIBMITTELGEMISCH
MELANGE PROPULSEUR PRATIQUEMENT AU HYDRO SOUS PRESSION

(30) Priority: 19.05.2005 IT MI20050915
(43) Date of publication of application: 02.04.2008
(73) Proprietor: THERAPICON SRL, I-20146 Milan (IT)
(72) Inventor: VERONESI, Paolo Alberto, I-20146 Milan (IT); VERONESI, Anna Maria, I-20146 Milan (IT); RODRIGUEZ, Pablo EA, Cordoba, 5000 (AR)
(74) Representative: Colombo, Stefano Paolo
(86) International application number: PCT/EP2006/062242
(87) International publication number: WO 2006/122905

(56) References cited:
- WO-A-94/14408
- WO-A2-02/30383
- GB-A- 1 190 013
- GB-A- 1 597 147
- US-A1- 2003 007 929
- US-A1- 2005 042 182

## Description

The invention relates to a pressurized propellant mixture made from a pressurized substantially water-free homogeneous solution of inert ingredients. Of particular interest is the use of the pressurized propellant mixture in a pressurized pharmaceutical fill composition. Such a composition is formed by combining the pressurized propellant mixture with at least one therapeutically effective agent. The resulting pharmaceutical pressurized fill composition is intended for topical administration in a wide range of applications for human and/or animal use, where the therapeutically effective agent may act locally or transdermally.

The special pressurized propellant mixture of the invention exerts the simultaneous functions of propellant, carrier, preserving agent, disinfectant and stabilizer. Preferred embodiments of the invention are pressurized pharmaceutical fill compositions containing at least one therapeutically effective agent useful in treatment of burns, wounds healing, abrasions, skin delayed healing conditions and of fungal, bacterial and viral cutaneous infections. Other additional preferred embodiments are the pressurized pharmaceutical fill compositions containing at least one therapeutically effective agent intended to elicit antiacneic, antiseborrheic, keratolytic, antiandrogenic, analgesic, anaesthetic, anti-inflammatory, antiallergic and antihistaminic therapeutic effects.

Among the pathological skin conditions, healing of wounds is nowadays one of the major therapeutic problems. Wounds are internal or external bodily injuries or lesions caused by physical means, such as mechanical, chemical, viral, bacterial, which disrupt the normal continuity of the structures. Such bodily injuries include contusions, wounds, in which the skin is unbroken, incision, wounds in which the skin is broken by a cutting instrument, and lacerations, wounds in which the skin is broken by a dull or blunt instrument. Wounds may be caused by accidents or by surgical procedures, as for instance complicated dehiscent and infected wounds. In other cases wounds are resulting from metabolic or circulation disorders (diabetic foot and leg ulcers), often linked to simultaneous mechanical pressure problems, as for instance in the case of pressure sores (bedsores).

A burn is one of the most traumatic and severe injuries that the human body can sustain. The major problem is that even if the burn itself does not prove fatal, the infection that follows might. A severe thermal burn removes the upper protective layers of the dermis and epidermis and disrupt the immunological barriers to infections. This retards the regrowth of normal skin and promotes collagen formation which leads to scarring.

Wound and burn healing consists in a series of processes whereby injured tissue is repaired, specialized tissue is regenerated and new tissue is reorganized. The healing process consists of the three major phases : (a) an inflammation phase (0-72 hours), (b) a cellular proliferation phase (3-15 days) and (c) a remodelling phase (4 days-6 months or over).

During the inflammation phase, platelet aggregation and clotting form a matrix which traps plasma proteins and blood cells to induce the influx of various type of cells. During the cellular proliferation phase, new connective or granulation tissue and blood vessels are formed. During the remodelling phase, granulation tissue is replaced by a network of collagen and elastin fibers leading to the formation of scar tissue. When cells are injured or killed as a result of a wound from a different cause, a wound healing process is required to produce new cells to replace the dead ones. Therefore, the healing process requires the immediate suppression or attenuation of the cause, the reversal of cytotoxicity, the reduction of inflammation, and the stimulation of the cellular viability and proliferation. During the initial stages of healing, wounds require low levels of oxygen to reduce or suppress the oxidative damage, but they need higher levels of oxygen in the later stages of healing to promote collagen formation by the fibroblasts; occlusive systems are not, therefore, recommended.

Several treatments have been described in literature to elicit healing processes in wounds, burns and abrasions, but the desirable effects are often physiologically neutralized by induced opposite mechanisms. In fact, mammalian cells are continuously exposed to activated oxygen species, such as superoxide, hydrogen peroxide, hydroxy radical, oxygen and these reactive oxygen intermediates are generated in vivo by cells in response to aerobic metabolism, catabolism of drugs and other xenobiototics, ultraviolet and x-ray radiation, and the respiratory burst of phagocytic cells (such as white blood cells to kill invading bacteria such as those introduced through wounds or burns. Hydrogen peroxide, for example, is produced during respiration of most living organisms, but especially by stressed and injured cells, but it is renowned that hydrogen peroxide and these oxygen species can injure cells. A typical case is lipid peroxidation which plays a positive effect in the oxidative degradation of unsaturated lipids, but is highly detrimental to membrane structure and function, causing also numerous cytopathological effects. Cells defend against lipid peroxidation by producing radical scavengers such as superoxido dismutase, catalase and peroxidase, but injured cells have a substantially decreased ability to produce radical scavengers. An excess of hydrogen peroxide can react with DNA to cause backbone breakage, produce mutations and alter and liberate bases. Such oxidative biochemical injury can result in the loss of cellular membrane integrity, reduces enzyme activity, changes in transport kinetics, changes in membrane lipid content, and leakage of potassium ions, amino acids, and other cellular material.

Therefore, attempts made during the past to use antioxidants to inhibit damage associated with active oxygen species, such as, for instance, pyruvate and other alpha-ketoacids, alpha-hydroxyacids and aliphatic compounds as pyruvic acid and lactic acid (U.S. Pat. Nos. 3,920,835, 3,984,556 and 3,988,470). Other authors have disclosed compounds, methods, compositions consisting of amides and ammonium salts of alpha-hydroxyacids, beta-hydroxyacids and alpha-ketoacids (U.S. Pat. Nos. 4,105,783, 4,197,316 and 4,294,852).

Again lactate and sodium pyruvate alone or in mixture were reported to reduce the number of erosions and ulcers (Pushmann, Arzneimittel Forschung, 33, pp 410-413 and 415-416 (1983). Many other antioxidants, alike tocopherol (U.S. Pat. 4,847,071), a mixture of amino acids, minerals and lipophylic vitamins (U.S. Pat No. 4,533,637) and burns compositions containing silver sulfadiazine have also been described (U.S. Pat. Nos. 3,751,590 and 5,143,717).

A longer introduction has been dedicated to burns and wounds healing problems, since the repair mechanism is almost the same once the primary cause of the pathological degeneration has been removed. Often these degenerative dermatological processes are caused by fungi, bacteria or viruses, so that in those cases a specific therapeutically effective active shall be included in the pharmaceutical composition to be used. Nowadays, other increasing pathologies are affecting the dermis and epidermis in the population of the more developed countries, mainly due to the effects of the stress or of the environmental pollution, alike acne, excessive seborrhea and keratogenesis, impaired hair growth, also requiring specific treatments. Similarly, there is also an increasing demand for analgesics, anaesthetics, and non-steroidal anti-inflammatory drugs to treat inflammatory or painful conditions of some particularly affected areas of the human body, so that a local treatment is preferred to a systemic treatment for its inferior incidence of side effects of the used drugs. However, due to the indirect and direct aggression of thousands of new rather aggressive chemical products which have massively entered in the human life cycle, there is an increasing use of topical steroids, and of antiallergic and antihistaminic medicinal preparations.

The treatment of the above pathological conditions very often requires the topical application of the intended therapeutically effective active agent, which shall be uniformly sprayed on the affected areas of the body, sometimes for long periods. Many delivery systems for the topical application of a pharmaceutical active ingredient are currently available and include lotions, creams, gels, ointments, pastes, foams, medicated dressing or patches, nebulizing systems or sprays. The nebulizing devices are generally a combination of a glass or plastic container, where is placed the medicinal product to be nebulized. The nebulization is achieved by means of medicinal product to be nebulized. The nebulization is achieved by means of a manually operating pump (not pressurized), which is co-operating with a dispensing applicator, and many types of these devices are already available in the market. By contrast, the pressurized spray devices consist of a glass bottle or preferably of an aluminium can, which contains the medicinal composition to be sprayed. Many containers are available that have been anodized. They also may have an internal coating made from an epoxy-type resin. On the upper part of the container there is usually a 20-30 mm opening, so as to receive the standard valves. The valve is reciprocating with a dispensing nozzle or applicator, presenting a normal or a special shape and size design. Many types of these nozzles or applicators are currently available on the market.

A number of existing aerosol compositions include the following. US2005/0042182 discloses pharmaceutical, cosmetic and cosmeceutical foamable compositions for topical application, containing, as an active ingredient, urea and/or a derivative thereof, processes of manufacturing these compositions and uses of these compositions in the treatment of various dermatological conditions such as, for example, conditions associated with dry skin and/or scalp.

WO02/30383 discloses a deodorant product for the benefit of the human body or articles worn in close proximity thereto. This invention involves the application of an anti-microbial product comprising a transition metal chelator and a phenolic or enolic compound that is (a) a transferrin dissociation promoter that operates by aiding the reduction of iron(III) bound to transferrin to iron(II) and/or an anti-oxidant comprising a tert-butylphenol group.

WO94/14408 discloses deodorant compositions for topical applications to human skin, for example, underarm, containing as an active agent a layered hydroxide which contains copper and at least one other metal, and has the crystal structure of spangolite.

GB1597147 relates to a self-propelled or aerosol dispensing container including a vapour phase or propellant and a liquid phase containing the propellant, in liquid form, as well as a liquid to be dispersed. The aerosol comprises 3.5% aluminium chlorohydrate (as an antibacterial), 6.14% isopropylmyristate and 90% isobutane.

GB1190013 disposes an aerosol spray composition for use in feminine hygiene for application to the vaginal area comprising (a) a highly volatile aerosol propellant or propellant mixture (at least 50%); (b) at least 0.1% (-10%) by weight of an emollient substance or mixture of substances selected from the group consisting of (i) fats and oils and (ii) oily and fatty non-ionic emulsifying agents and (c) a polymeric bactericide (1-6%).

US2003/0007929 discloses a topical spray composition of corticosteroid, an alcohol, a propellant and isopropyl myristate.

However, at least four main factors shall be carefully considered when adopting a delivery system for dispensing a pharmaceutical compositions intended for the treatment of any of the above pathological conditions. Firstly, it depends upon the pharmacokinetic characteristics of the therapeutically effective agent, as for instance whether only a topical or a systemic medicinal effect is desired. Some hormonal drugs require a transdermal absorption in order to obtain the desired systemic levels, so that also a strong absorption enhancer shall be included in the composition. By contrast, when a topical anti-inflammatory property is desired, the preferred delivery system shall not enhance the systemic absorption of the active substance.

Secondly, the delivery system depends considerably upon the type of pathology to be treated and the extent of the affected area on which the pharmaceutical composition containing the intended therapeutically active agent is to be applied. For instance, creams, ointments (having a fatty or oily appearance and touch) and patches are not preferred when they are intended to be applied on visible skin portions, such as the face, neck, arms, knees and legs. Therefore, not only must the pharmaceutical composition be therapeutically effective, but it must also meet the patient's requirements that it complies with certain cosmetic properties or features.

The third, but prevailing criteria of choice for a delivery system depends upon the severity, integrity and size of the cutaneous area to be treated. In case large and seriously affected areas have to be treated or when the medicinal product shall be applied without any mechanical trauma, as in the case of burns or wounds, a pressurized spray delivery system (aerosols) is the most eligible pharmaceutical form to assure uniform and atraumatic distribution of the medicinal product on the affected area.
The fourth important aspect is that the drug delivery system shall not be occlusive. In fact, the pharmaceutical composition shall not occlude the skin or the membrane breathing, this is in order to avoid irritation and allergic sensitization problems arising from prolonged exposure of the skin to the occlusion system.

Moreover it is desirable that the delivered pharmaceutical composition possibly also contains an antibacterial compound, comprising one or more ingredients with disinfectant, bacteriostatic, antimicrobial preservative and/or bactericidal properties, since its presence would offer the additional advantage to avoid the need to sterilize the composition by gamma-radiation at the end of the production cycle.

Finally it is recommended that the pressurized spray delivery system shall also contain a suitable preserving and stabilizing agent to ensure the protection of the pharmaceutical composition from bacterial proliferation, without the necessity to sterilize the finished product with gamma radiations or with other extreme physical practices, which shall be avoided, if possible, for medicinal products.

### BRIEF DESCRIPTION

Preferably the invention provides a pressurized propellant mixture. It is a further preferred that the invention provides a pressurized pharmaceutical fill composition resulting from the admixture of at least one sprayable therapeutically effective agent and the pressurized propellant mixture. The pressurized pharmaceutical fill composition is particularly suitable for spraying as an aerosol form of such therapeutic agent for topical administration.

It is further preferred that the special pressurized propellant mixture of the invention, despite it containing very few constituents, exerts several simultaneous functions such as propellant, carrier, preserving agent, disinfectant and stabilizer, which are all desired for the delivery of the therapeutically effective agent.

It is another preference of the present invention to provide a pressurized propellant mixture consisting of a substantially water-free homogeneous solution of inactive ingredients, in which at least one therapeutically effective agent is uniformly dissolved, suspended or emulsified.
It is a main object of the invention that the pressurized pharmaceutical fill composition contains, additionally, at least one therapeutically effective agent useful for the topical treatment in the pharmaceutical form of aerosol of some pathological conditions affecting the dermis and epidermis, and presenting difficulties of management such as burns, wounds healing, abrasions, skin delayed healing conditions, fungal, bacterial and viral topical infections. It is a further object of the invention to provide a pressurized pharmaceutical fill composition, suitable to be sprayed as topical aerosol, containing at least one therapeutically effective agent intended to exert antiacneic, antiseborrheic, keratolytic, antiandrogenic, analgesic, anesthetic, anti-inflammatory, antiallergic and antihistaminic therapeutic properties.

It is further preferred to provide an improved pharmaceutical pressurized fill composition in the form of aerosol that may be easily, quickly and relatively painlessly sprayed topically to burns, wounds, abrasions and skin delayed healing conditions. For these specific uses where a sterile product is required to avoid the risk to induce infections, a further advantage is represented by the presence of an antibacterial compound which comprises one or more ingredients with disinfectant, bacteriostatic, bactericidal and/or antimicrobial preservative properties, thus avoiding also the final sterilization of the product by gamma-radiation.

Its is an additional preference of the invention to provide a pharmaceutical pressurized fill composition in the form of aerosol that may be suitably, conveniently and uniformly sprayed topically to extensive skin portions as in such case when an analgesic, anaesthetic, anti-inflammatory, antiallergic, antihistaminic therapeutic effect is desired.

It is yet a further preference of the invention to provide a pharmaceutical pressurized fill composition in the form of aerosol to be conveniently applied to uncovered parts without an oily aspect as in such case when an antiacneic, antiseborrheic, keratolytic and antiandrogenic therapeutic affect is desired.

Another technical preference of the invention is to provide a substantially water-free pharmaceutical pressurized fill composition, in order to minimize the possibility of interaction between the ingredients, to avoid the frequently reported instability of several active ingredient in presence of moisture, and finally to yield composition which are conveniently stable during the storage period comprised between their production and subsequent use. Preferably, the pressurized propellant mixture is completely free of water.

It is a physiological preference of the invention to provide a pharmaceutical pressurized fill composition physiologically compatible with the skin conditions (neither excessively acidic nor basic), so as neither to cause nor to exacerbate the inflammation or the irritation of the treated skin.

Similarly, it is another physiological preference of the invention to provide a pharmaceutical pressurized fill composition that after spraying it on the affected skin portion, shows breathable characteristics and not cause an occlusive film.

According to one embodiment of the present invention there is provided a substantially water-free pressurized propellant mixture comprising a homogeneous solution of few inert ingredients. The present invention may also provide a pharmaceutical pressurized fill composition resulting from the admixture (solution, suspension or emulsion) of at least one sprayable therapeutically effective agent and the substantially water-free pressurized propellant mixture, the therapeutically effective agent being suitably elected in respect of the intended pathological condition to be treated by topical route.

Said substantially water-free pressurized propellant mixture contains (1) at least one liquid inert propellant, but preferably a pressure-liquefied mixture of at least two aliphatic hydrocarbons, under a suitable pressure conditions and in a convenient ratio with the other inactive ingredients; (2) at least one inert physiologically acceptable carrier (preferably, medium chain fatty acids esters), but preferably a mixture of at least two inert carriers, which shall be soluble in the pressure-liquefied mixture, in a convenient ratio with the ingredient at point (1) and with the intended amount of therapeutically effective agent; (3) at least one antibacterial compound preferably comprising one or more ingredients with bactericidal, disinfectant, bacteriostatic and/or antimicrobial preservative properties (preferably, one or more substituted chlorophenols), which shall be also soluble in the pressure-liquefied mixture and in the carrier, at a convenient concentration; and (4) optionally (4a) a physiologically compatible emollient-humectant and (4b) a suitable amount of an organic solvent selected among the group of low chain alcohols, when an organic solvent is required to solubilize the therapeutically effective agent.

The pharmaceutical fill composition may be aerosolized at a specific pressure in very different packs already available on the market in respect of their application properties. The packed pharmaceutical fill composition may comprise a pressure container (from glass or metal, preferably from aluminium) fitted with a valve construction suitable for discharging the filling material present and sealed inside the container. The pharmaceutical fill composition can be of very diverse nature, depending primarily on the used therapeutically effective agent and on the selected quantities of each inert ingredient.

### DESCRIPTION OF PREFERRED EMBODIMENTS

Tlierefore, in one embodiment the invention provides a substantially water-free pressurized propellant mixture comprising a homogeneous solution of
a) at least 80.0% by weight of at least one pressure liquefied propellant;
b) up to 15.0% by weight of at least one carrier;
c) from 0.01 % to 1.0% by weight of at least one bactericidal compound; optionally
d) from 0.5% to 5.0% by weight of at least one emollient-humectant; and optionally
e) less than 5.0% by weight of at least one organic solvent.

Preferably, the at least one pressure-liquefied propellant is a mixture in the same or different ratio of at least two aliphatic hydrocarbons selected among n-butane, i-butane, propane, under variable suitable pressure conditions, representing substantially at least 80.0 % by weight, more preferably 90.0 % by weight. Preferably, the at least one carrier is physiologically acceptable, for example, isopropyl myristate or isopropyl palmitate or a variable mixture thereof, purposely selected for their solubility in the pressure-liquefied propellant mixture, and representing not more than 15.0 % by weight, more preferably about 5.0 % by weight. The at least one bactericidal compound, preferably one or more ingredients with disinfectant, bactereostatic, bactericidal and/or antimicrobial preservative properties, and is preferably selected among chlorocresol or chloroxylenol or a mixture thereof in the same or a different ratio, in a variable global range of from 0.001 % to 1.0 % by weight, more preferably from 0.005 to 0.1 % by weight. The optional at least one physiologically compatible emollient-humectant is, preferably cholesterol or any other softening agent physiologically and chemically compatible with the pressurized propellant mixture, in a variable range of from 0.5 % to 5.0% by weight, more preferably about 3.0 % by weight; and the optional at least one organic solvent is selected among the group of low chain alcohols (anhydrous), included in the composition when its presence is required to better solubilize the therapeutically effective agent, which shall be physiologically and chemically compatible with the pressurized propellant mixture, and representing less than the 5.0 % by weight, preferably about 3.0 % by weight.

The present invention may also provide a pressurized pharmaceutical fill composition comprising a) a water-free pressurized propellant mixture as described above, and b) from 0.001 % to 5.0% by weight of at least one therapeutically effective agent or a mixture thereof, selected in accord with the intended therapeutic use, suitably solubilized, suspended or emulsified in said pressurized propellant mixture. Preferably, the therapeutically effective agent or a mixture thereof is sprayable and is dosed at a therapeutically effective amount in variable concentrations ranging from 0.001 % to 5.0 % by weight, more preferably 1.0 % by weight. A detailed list of therapeutically effective agents (grouped by therapeutic class), which can be delivered on the skin surface to achieve the desired effect will be further available in this part of the invention.

The present invention may also provide an aerosol dispenser containing a pressurized pharmaceutical fill composition as described above, and also a method of filling such an aerosol dispenser with the pharmaceutical fill composition. The method of filling an aerosol dispenser comprises the steps of a) filling a suitable container with the desired weight or volume of a carrier, a antibacterial compound, a therapeutically effective agent, and optionally, an emollient and an organic solvent; b) sealing the filled container with a pressure valve system; and c) further filling the container with a pressure liquefied propellant.

The foregoing and other objects, features and advantages of the invention will be apparent from the following more particular description of the preferred embodiments of the invention.

In a preferred embodiment of the invention the pressurized propellant mixture may result from a precise combination of few selected etherogenous inert ingredients exerting different functions and forming an homogeneous pressurized liquid solution for the delivery of a therapeutically effective agent, so that the pharmaceutical pressurized fill composition does not need to be shaken again before its further use even after prolonged standing. The above mentioned pressurized propellant mixture for pharmaceutical compositions is a suitable inert media fill to deliver aerosols of a therapeutically effective agent, thus representing a significant advantage over the state of the art.

In fact, the first technical feature is that at least one pressure-liquefied propellant or preferably a variable mixture in a different ratio of at least two aliphatic hydrocarbons thereof, selected among n-butane, i-butane and propane, are used in such an amount that the pressure-liquefied propellant is representing not less than 80.0 % by weight. However, firstly the aliphatic hydrocarbons are completely inert, so that they react neither with the therapeutically effective agent nor with the other ingredients.

Secondly, the pressurized propellant mixture is substantially water-free, so that the therapeutically effective agent and also the final pharmaceutical pressurized fill composition may result more stable in an anhydrous media. Preferably, the propellant mixture is substantially free of water, since many drug substances used in medicine are highly sensitive or may even easily degrade in presence of water or even of moisture.
Therefore the resulting aerosols of pharmaceutical pressurized fill composition are substantially water- or moisture-free, or preferably, completely water- or moisture-free.

In another preferred technical feature, despite a certain safety hazard associated with the flammable liquid gases, the pressure-liquefied propellant mixture presents significant ecological advantages over the CFC (chlorofluorocarbons), since the publication of the ozone theory is indicating that CFC and other chlorine-containing organic compounds degrade ozone.

The preferred aliphatic hydrocarbons are those having a liquid density comprised from 1.5 g/cm³ to 3.0 g/cm³, but their ratio may vary according to several factors, mainly the cumulative mass of the rest of the media fill. Typical pressure-liquefied propellant mixtures for the anhydrous pharmaceutical aerosols of the invention are shown in the following table:

| | mixture 1 | mixture 2 | mixture 3 | mixture 4 |
|---|---|---|---|---|
| n-butane | 40.0 % | 2.0 % | 5.0 % | 5.0 % |
| isobutane | --- % | 80.0 % | 75.0 % | 70.0 % |
| propane | 60.0 % | 18.0 % | 20.0 % | 25.0 % |

| | | | | |
|---|---|---|---|---|
| Gases are expressed as approximate volume %. | | | | |

In a second preferred technical feature the physiologically acceptable carrier has been purposely selected among isopropyl myristate and isopropyl palmitate or a mixture thereof for the remarkable and useful solubility at the used concentration (substantially less than 15.0 % by weight) in the pressure-liquefied mixture of aliphatic hydrocarbons. In another technical feature the preferred concentrations of isopropyl myristate and isopropyl palmitate or a mixture thereof are typically in the range of from 1.5 % to 4.0 % by weight, but also concentrations of approximately 5.0 % by weight or even up to about 10.0 % may be used, when these masses are required to dissolve a large amount of therapeutically active ingredient. Moreover the selected pharmaceutically acceptable carrier exhibits additional advantages over the current excipients for topical compositions. In fact, in another technical feature it has been observed by the authors that many therapeutically effective agents are conveniently soluble in these selected medium chain fatty acids esters; moreover they are nongreasy emollients that are absorbed readily by the skin. In addition, due to their ready absorption, the selected carrier is skin "breathable" and non occlusive. Moreover the selected carrier shows a good stability, is compatible with the other inactive and active ingredients of the pharmaceutical composition, and is safe for pharmaceutical formulations.

An additional technical feature is that the selected chlorophenols, such as chlorocresol or chloroxylenol or a mixture thereof, have been typically selected and qualified as an essential ingredient of the pressurized propellant mixture for exerting at the used concentration ranges of from 0.01 % to 1.0 % by weight two peculiar properties : bactericidal-disinfectant and also antimicrobial preservative. The preferred concentration is generally of about 0.1 % by weight, but due to its characteristic phenolic odour a concentration above 0.2 % is not used very often. Moreover, chlorocresol or chloroxylenol have been purposely selected for its solubility at the used concentration (generally about 0.1 % by weight) in the carrier and in the pressure-liquefied propellant, so that an homogeneous solution mixture is obtained. In addition chlorocresol or chloroxylenol at the used concentrations shows a significant preservative efficacy, so that it is very useful to control the microbial stability of the final pharmaceutical pressurized fill composition of the invention. The bactericidal activity is against both Gram positive and Gram negative organisms (including Pseudomonas aeruginosa), spores, moulds, and yeasts. It is most active in acidic solutions and shows synergic antimicrobial effects with other antimicrobial agents. Chlorocresol or chloroxylenol is suitable for controlling bacteria (bactericidal at concentrations of approximately 0.08 % with a contact of 10 minutes, being a typical MIC 0.02 %), fungi (from 0.01 to 0.04 % after 24 hours contact), and spores (at a concentration greater than 0.012 % at 80° C).

Therefore in another basic preferred embodiment of the invention, the pressurized propellant mixture, which constitutes the inert support of the pharmaceutical composition, has been purposely designed to express multiple combined functions as those of propellant, carrier, antimicrobial preservative, bactericidal disinfectant and stabilizer for one or more therapeutically effective agent to be combined therewith.

Furthermore the presence of the bactericidal compound, for example, chlorocresol or chloroxylenol or a mixture thereof, in the pressurized propellant mixture is essential and results of great importance to control the microbiological content and to assure the sterilization of the pharmaceutical pressurized fill composition, thus avoiding to sterilize by gamma-radiation the aerosol dispenser at the end of its production process.

In another optional feature of the invention, when a final pharmaceutical composition with more softening characteristics is required, the pressurized propellant mixture may also optionally contain at least an emollient-humectant ingredient. The preferred substance is cholesterol, which may be used in a variable range of from 0.5 % to 5.0 % by weight.

In another additional optional feature of the invention, at least one organic solvent selected among the group of anhydrous low chain alcohols may be included in the pressurized propellant mixture when its presence is desirable to enhance the solubility of a specific therapeutically effective agent. The typically preferred alcohols are anhydrous ethanol and isopropanol or a variable mixture thereof, and the used quantity shall not exceed 5.0 % by weight, but exceptionally also up to 10.0% by weight.

In the main characterizing embodiment of the invention at least one sprayable therapeutically effective agent or a mixture thereof, selected in accord with the intended therapeutic use, is solubilized, suspended or emulsified in said pressurized propellant mixture.

In another preferred embodiment of the invention the selected pharmaceutical effective agent is typically dosed in a therapeutically effective amount in accord with its intended therapeutic use, so that they are suitably solubilized, suspended or emulsified in said pressurized propellant mixture at variable concentration of from 0.001 % up to about 5.0 % by weight. Occasionally, higher concentrations up to 5.0 % or 10.0 % by weight may be used.

A wide range of embodiments may be achieved for the available therapeutically effective agents, which may be conveniently compounded in the pharmaceutical fill composition (pharmaceutical aerosols) of the invention, so that they have been better grouped in accord with their known therapeutic effect. Indeed the present invention is intended to encompass and to be suitable for use by substituting any of the following drugs as therapeutically effective agent in the pharmaceutical pressurized fill composition of the invention :

### A-Embodiment for Burns, Wounds, Abrasions, and Delayed Skin Healing Conditions Topical Treatments

Accordingly, in a preferred embodiment at least one therapeutically effective agent useful in treatment of burns, wounds, abrasions and associated skin pathological conditions, is selected from the group of anabolic or androgen substances, which may enhance the cellular proliferation and remodelling rates. Among such anabolic substances which can stimulate the burns and wounds healing process, authors have discovered that some molecules and some of their esters thereof are typically able to enhance the cell recovery, as for instance androisoxazole, androstenediol, bolandiol, bolasterone, clostebol, ethylestrenol, formebolone, methandriol, methenolone, methyltrienolone, nandrolone, norbolethone, oxabolone, oxymesterone, quinbolone, stenbolone, and trenbolone. Also many other androgen substances and some of their esters thereof are able to stimulate the burns-and wounds-healing process, as for instance boldenone, fluoxymesterone, mestanolone, mesterolone, oxandrolone, oxymetholone, prasterone, stanlolone, stanozolol, testosterone, tiomesterone.

The inventors have also surprisingly found that by combining said healing promoters with an antibacterial agent, which can prevent or treat the local bacterial infections, the burns or wound healing composition can improve the resuscitation rate of the injured cells and the proliferation rate on new mammalian cells to replace the dead cells. Such healing combination may remarkably reduce the size, duration, and severity of potentially infectable or of infected burns or wounds. The antibacterial agents which may be typically employed in combination with the anabolic or androgen agent, may be selected from a wide variety and shall be compatible and do not react with the "pressurized propellant mixture".

Nonlimiting illustrative specific examples of antibacterial agents include aminoglycosides, macrolides, lincosamides, polypeptides, penicillins, tetracyclines, amphenicols, ansamycins and other antibiotics or standard mixtures thereof or any pharmaceutically acceptable addition salts thereof.

Preferably, the antibacterial agent is typically selected from the group of antibiotics, which include the amynoglycosides, such as amikacin, apramycin, arbekacin, bambermycins, butirosin, dibekacin, dihydrostreptomycin, fortimicin(s), fradiomycin, gentamicin, ispamicin, kanamycin, micronomicin, neomycin, neomycin undecylenate, netilmicin, paromomycin (aminoxidin), ribostamycin, sisomicin, spectinomycin, streptomycin, tobramycin, trospectomycin; macrolides-lincosamides such as erytromycin, clindamycin, lincomycin; polypeptides such as amphomycin, bacitracin, capreomycin, colistin, enduracidin, enviomycin, fusafungine, gramicidin(s), gramicidin S, lysozyme, mikamycin, polymyxin, pristinamycin, ristocetin, teicoplanin, thiostrepton, tuberactinomycin, tyrocidine, tyrothricin, vancomycin, viomycin(s), virginiamycin, zinc bacitracin; penicillins such as amoxicillin, ampicillin, apalcillin, bacampicillin, benzylpenicillin, carbenicillin, fenbenicillin, hetacillin, metampicillin, methicillin, mezlocillin, nafacillin, oxacillin, piperacillin, pivapicillin, sultamicillin, talampicillin, temocillin, ticarcillin; tetracyclines such as apicycline, chlortetracycline, clomocycline, demeclocycline, doxycycline, guamecycline, lymecycline, meclocycline, methacycline, minocycline, oxytetracycline, penimepicycline, rolitetracycline, sancycline, tetracycline; amphenicols such as azidamfenicol, chloramphenicol, florfenicol, thiamphenicol; ansamycins such as rifabutine, rifamide, rifampicin, rifamycin, rifapentine; and other antibiotics, such as cycloserine, mupirocin, tuberin or standardized mixtures as "neosporin" (a mixture of polymyxin B sulfate, bacitracin zinc and neomycin sulfate), and any pharmaceutically acceptable addition salts thereof.

The one or more antibacterial agents to be associated with the healing enhancer of the present invention may be used in the therapeutically effective dose for topical use, as well known in the pharmaceutical art. In fact, the amount of antibacterial agent which may be employed in the antibacterial burns- or wounds- healing therapeutic compositions of the present invention may vary depending upon the therapeutic dosage recommended or permitted for the particular antibacterial agent. In general, the amount of antibacterial agent present is the ordinary dosage intended to elicit and to obtain the desired therapeutic result. Such dosages are known to the pharmaceutical skilled artisans or practitioner in the medical arts and are not part or claimed in the present invention. In a preferred embodiment the antibacterial agent in the antibacterial burns- and wounds- healing composition is present in an amount from 0.02 % to 5.0 %, preferably from 0.15 % to 2.0 % by weight, this depending mainly on the specific chemical structure and individual MIC (Minimum Inhibitory Concentration) of each considered antibacterial agent.

Similarly, in a preferred embodiment, the healing enhancer in the composition may vary from 0.05% to 2.0% by weight, preferably from 0.1 % to 1.0% by weight.

### B-Embodiment for Topical Antifungal (antibiotic/synthetic) Treatments

In another preferred embodiment of the invention one therapeutically effective agent useful in treatment of fungine infections, is represented by an antifungal antibiotic from the group of polyenes such as amphotericin-B, candicidin, dermostatin, filipin, fungichromin, hachimycin, hamycin, lucensomycin, mepartricin, natamycin, nystatin, pecilocin, perimycin or other related antibiotic derivative such as azaserine, griseofulvin, oligomycins, neomycin undecylenate, siccanin, tubercidin, viridin, while another group is represented by a synthetic antifungal from the group of allylamines such as butenafine, naftifine and terbinafine; imidazoles such as bifonazole, butoconazole, chlordantoin, chlormidazole, cloconazole, clotrimazole, econazole, enilconazole, fenticonazole, flutrimazole, isoconazole, ketoconazole, lanoconazole, miconazole, omoconazole, oxiconazole nitrate, sertaconazole, sulconazole, tioconazole; triazoles such as fluconazole, itraconazole, saperconazole, terconazole; other antifungals such as acrisorcin, amorolfine, biphenamine, bromosalicylchloranilide, buclosamide, calcium propionate, chlophenesin, ciclopirox, coparaffinate, diamthazole, flucytosine, hexetidine, loflucarban, nifuratel, potassium iodide, propionic acid, pyrithione, salicylanilide, sulbentine, tenonitrozole, triacetin, undecylenic acid or any pharmaceutically acceptable addition salts thereof.

In general, the amount of antifungal agent present is the ordinary dosage intended to elicit and to obtain the desired therapeutical result. Such dosages are known to the pharmaceutical skilled artisans or practitioner in the medical arts and are not part or claimed in the present invention. The antifungal agent in the composition is typically present in an amount from 0.1 % to 3.0 %, preferably from 1.0 % to 2.0 % by weight, this depending mainly on the specific individual antifungal activity of the therapeutically effective agent.

### C-Embodiment for Topical Antiseptic Treatments

In an additional preferred embodiment one therapeutically effective agent useful in treatment of topical bacterial infections, is represented by an antiseptic agent selected from the group of guanidines such as alexidine, ambazone, chlorhexidine, picloxydine; halogens/halogen compounds such as bismuth iodide oxide, bismuth iodosubgallate, bismuth tribromophenate, bornyl chloride, calcium iodate, iodic acid, iodine, iodine monochloride, iodine trichloride, iodoform, methenamine tetraiodine, povidone-iodine, sodium hypochlorite, sodium iodate, triclocarban, triclosan, troclosene potassium; nitrofurans such as furazolidone, nidroxyzone, nifuroxime, nifurzide, nitrofurazone; phenols such as acetomeroctol, bithionol, cadmium salicylate, carvacrol, chloroxylenol, clorophene, creosote, cresol, fenticlor, hexachlorophene, 1-napthyl salicylate, 2-napthyl salicylate, 2,4,6-tribromo-m-cresol, 3',4',5-trichlorosalicylanilide; quinolines such as aminoquinuride, benzoxiquine, broxyquinoline, chloroxine, chlorquinaldol, cloxyquin, ethylhydrocupreine, euprocin, halquinol, hydrastine, 8-hydroxquinoline sulfate, iodochlorhydroxyquin; others such as aluminum acetate solution, aluminum subacetate solution, aluminum sulfate, 3-amino-4-hydroxybutyric acid, boric acid, chlorhexidine, chloroazodin, m-cresyl acetate, cupric sulfate, dibromopropamidine, ichthammol, negatol, noxythiolin, octenidine, ornidazole, beta-propriolactone, alpha-terpineol or any pharmaceutically acceptable addition salts thereof.

In general, the amount of antiseptic agent present is the ordinary dosage intended to elicit and to obtain the desired antiseptic result. Such dosages, known to the pharmaceutical skilled artisans or medical practitioners in the medical arts, are present in the antiseptic composition in an amount from 0.05 % to 5.0 %, preferably from 0.5 % to 2.0 % by weight, this depending mainly on the specific individual antiseptic activity of the therapeutically effective agent.

### D-Embodiment for Topical Antiviral Treatments

In a further preferred embodiment one therapeutically effective agent, selected among those useful in treatment of topical viral infections, is represented by an antiviral agent selected from the group of purines/pyramidinones such as acyclovir, acyclovir prodrug, cidifovir, cytarabine, dideoxyadenosine, didanosine, edoxudine, famcyclovir, floxuridine, gancyclovir, idoxuridine, inosine pranobex, n-docosanol, lamivudine, pencyclovir, sorivudine, stavudine, trifluridine, velacyclovir, zalcitabine, zidovudine; other such as acemannan, acetylleucine, monoethanolamine, amantadine, amidinomycin, delaviridine, foscarnet sodium, indinavir, kethoxal, methysazone, moroxydine, nevirapine, podophyllotoxin, ribavirin, rimantadine, ritonavir, saquinavir, stallimycin, tromantadine, xenazoic acid or any pharmaceutically acceptable addition salts thereof.

The amount of antiviral agent present is the ordinary dosage intended to elicit and to obtain the desired antiviral result. These dosages, known to the pharmaceutical skilled artisans or medical practitioners in the medical arts, are present in the antiviral composition in an amount from 0.3 % to 5.0 %, preferably from 1.0 % to 2.0 % by weight, this depending mainly on the specific individual antiviral activity of the therapeutically effective agent.

### E-Embodiment for Topical Antiacne. Antiseborrheic and Keratolytic Treatments

In another additional embodiment one therapeutically effective agent, selected among those useful in treatment of acne, is represented by an active substance such as algestone acetophenide, azelaic acid, benzoyl peroxide, cioteronel, cyproterone, isotretinoin, motretinide, resorcinol, retinoic acid, tazarotene, tetroquinone, tioxolone, tretinoin or any pharmaceutically acceptable esters or addition salts thereof, while in treatment of seborrhea the therapeutically effective agent is represented by an active substance such as chloroxine, 3-o-lauroylpyridoxol diacetate, piroctone, pyrithione, resorcinol, selenium sulfides, tioxolone, or any pharmaceutically acceptable esters or addition salts thereof. For keratolytic treatments active substances such as alpha-hydroxy acids, glycollic acid and salycilic acid are used. For stronger keratolytic treatments, as required in psoriasis, active substances such as calcipotriol may be used.

The preferred quantity of the active substance useful in treatment of acne, seborrhea or keratitis in the composition of the invention is ranging from 0.02 % to 3.0 %, preferably from 0.10 % to 1.5 % by weight, this depending on the specific therapeutically effective agent used for the pathology to be treated.

### F- Embodiment for Topical Antiandrogen Treatments (Hair Growth)

In another preferred embodiment one therapeutically effective agent selected among those showing antiandrogen activity, useful for the treatment of hair growth, is represented by an active substance such as bicalutamide, bifluranol, cioteronel, cyproterone, delmadinone acetate, flutamide, nilutamide, osaterone, oxendolone or any pharmaceutically acceptable esters or addition salts thereof.

The amount of active substance in the composition of the invention in treatment of air growth may vary from 0.02 % to 3,0 %, preferably from 0.05 % to 1,0 % by weight, this depending on the specific pharmacological activity of the considered therapeutically effective agent.

### G-Embodiment for Topical Analgesic (non-narcotic) Treatments

In a further embodiment one pharmaceutically active ingredient with analgesic (non-narcotic) activity, selected among those useful to be formulated for the treatment of topical painful conditions, is represented by an active substance such as aceclofenac, acetaminophen, acetylsalicylsalicylic acid, benzydamine, bromofenac, bufexamac, capsaicin, clometacin, clonixin, diflunisal, fenoprofen, flufenamic acid, flupirtine, fluproquazone, flurbiprofen, gentisic acid, glafenine, ibufenac, indoprofen, ketoprofen, ketorolac, lornoxicam, loxoprofen, lysine acetylsalicylate, magnesium acetylsalicylate, metofoline, naproxen, nefopam, salicin, talniflumate, tenoxicam, tolfenamic acid, tramadol, zomepirac; or any pharmaceutically acceptable addition salts thereof.

The amount of analgesic (non-narcotic) is generally present in the ordinary dosage intended to elicit and to obtain the desired analgesic effect. Such dosages are known to the pharmaceutical skilled artisans or practitioner in the medical arts and are not part or claimed in the present invention. The quantity of analgesic (non-narcotic) is typically present in the composition of the invention in the range of from 0.5 % to 3.5 %, preferably of from 1.0 % to 2.0 % by weight, this depending mainly on the specific activity of each considered therapeutically effective agent and on the desired degree of analgesic activity.

### H-Embodiment for Topical Anaesthetic Treatments

In a further preferred embodiment one therapeutically effective agent selected among those useful to elicit an anaesthetic effect is represented by an active substance such as acetamidoeugenol, ambucaine, amethocaine, amylocaine, benoxinate, benzocaine, betoxycaine, bupivacaine, butacaine, butamben, butanilicaine, butethamine, carticaine, cinchocaine, etidocaine, dibucaine, dimethocaine, diperadon, etidocaine, etoxadrol, fomocaine, ketamine, lidocaine, mepivacaine, midazolam, phenacaine, piridocaine, prilocaine, procaine, propipocaine, propofol, tetracaine, thiopental, trimecaine, zolamine; or any pharmaceutically acceptable addition salts thereof.

Generally, the anaesthetic is present in the ordinary dosage intended to elicit and to obtain the desired anaesthetic effect. Such dosages are known to the pharmaceutical skilled artisans or practitioner in the medical arts and are not part or claimed in the present invention. The amount of anaesthetic suitable to elicit the desired effect is typically in the range of from 0.5 % to 4.0 %, preferably of from 1.0 % to 2.5 % by weight, this depending mainly on the specific anaesthetic activity. of the therapeutically effective agent.

### I- Embodiment for Topical Anti-inflammatory (NSAID) Treatments

Accordingly, in another further preferred embodiment one selected therapeutically effective agent useful in treatment of inflammatory topical conditions is represented by a non-steroidal anti-inflammatory drug (NSAID), such as etofenamate, flufenamic acid, isonixin, meclofenamic acid, mefanamic acid, niflumic acid, talniflumate, tolfenamic acid; arylacetic acid derivatives such as aceclofenac, acemetacin, alclofenac, amfenac, bromfenac, bufexamac, diclofenac, etodolac, fentiazac, glucametacin, ibufenac, oxametacine, pirazolac, proglumetacin, sulindac, tolmetin, zomepirac; arylbutyric acid derivatives such as fenbufen, xenbucin; arylcarboxylic acids such as clidanac, ketorolac, kinoridine; arylpropionic acid derivatives such as benoxaprofen, carprofen, fenoprofen, flunoxaprofen, flurbiprofen, ibuprofen, indoprofen, ketoprofen, loxoprofen, pirprofen, pranoprofen, suprofen, tiaprofenic acid; salicylic acid derivatives such as acethylsalycilic acid, diflunisal, gentisic acid, glycol salicylate, arginine and lysine acetylsalicylate, mesalamine, morpholine salicylate, olsalazine, sulfasalazine; thiazinecarboxamides such as ampiroxicam, droxicam, isoxicam, lornoxicam, piroxicam, tenoxicam; others such as S-adenosylmethionine, bendazac, benzydamine, guaiazulene, nabumetone, nimesulide, superoxide dismutase, tenidap; or any pharmaceutically acceptable addition salts thereof.

The amount of anti-inflammatory (non-steroidal) is present in the ordinary dosage intended to elicit and to obtain the desired anti-inflammatory effect. Such dosages are known to the pharmaceutical skilled artisans or practitioner in the medical arts and are not part or claimed in the present invention. The quantity of anti-inflammatory to elicit the desired effect is in a range of from 0.5 % to 10.0 %, preferably of from 1.0 % to 3.0 % by weight, this depending mainly on the specific individual pharmacological activity of the therapeutically effective agent.

### K - Embodiment for Topical Corticosteroids Treatments

In a further preferred embodiment one therapeutically effective agent useful in treatment of topical inflammatory conditions is represented by a cortocosteroid such as betamethasone, bethamethasone valerate, cortisone, dexamethazone, dexamethazone 21-phosphate, fludrocortisone, flumethasone, fluocinonide, fluocinonide desonide, fluocinolone, fluocinolone acetonide, fluocortolone, halcinonide, halopredone, hydrocortisone, hydrocortisone 17-valerate, hydrocortisone 17-butyrate, hydrocortisone 21-acetate, methylprednisolone, prednisolone, prednisolone 21-phosphate, prednisolone, triamcinolone, acetonide and some of their esters thereof. Further examples of steroidal antiinflammatory agents for use in the instant compositions include cortodoxone, fluoracetonide, fludrocortisone, difluorisone diacetate, flurandrenolone acetonide, amcinafel, amcinafide, beamethasone, chloroprednisone, clorcortelone, descinolone, desonide, dichlorisone, difluprednate, flucloronide, flunisolide, flucortolone, fluprednisolone, meprednisone, cortisone, hydrocortisone cyclopentylpropionate, flucetonide, fludrocortisone, flurandrenolone, medrysone, betamethasone benzoate, chloroprednisolone, clocortolone, descinolone acetonide, difluprednate, flucoronide, flumethasone, fluperolone, pramethasone, prednisolamate, prednival, cortivazol, formocortal, nivazol and some of their esters thereof.

The preferred quantity of the active substance useful in treatment of inflammatory skin conditions in the pharmaceutical composition of the invention is in the range of from 0.02 % to 3.0 %, preferably of from 0.10 % to 1.5 % by weight, this depending on the specific therapeutically effective agent used for the pathology to be treated.

### L-Embodiment for Topical Antiallergic or Antihistamic Treatments

In another additional preferred embodiment one therapeutically effective agent useful in treatment of allergic and antihistamic topical conditions is represented by an antiallergic substance such as amlexanox, astemizole, azelastine, cromolyn, ibudilast, lodoxamide, nedocromil, oxatomide, pemirolast, pentigetide, picumast, repirinast, suplast tosylate, tranilast, traxanox; or by an antihistamic substance selected from the group of alkylamine derivatives such as acrivastine, bamipine, brompheniramine, chlorpheniramine, dexchlorpheniramine, dimethindene, metron S, pheniramine, pyrrobutamine, thenaldine, tolpropamine, triprolidine; aminoalkyl ethers such as bietanautine, bromodiphenhydramine, carbinoxamine, clemastine, doxylamine, embramine, medrylamine, orphenadrine, phenyltoloxamine, setasine; ethylenediamine derivatives such as alloclamide, chloropyramine, chlorothen, histapyrrodine, methafurylene, methaphenilene, methapyrilene, pyrilamine, talastine, thenyldiamine, thonzylamine, tripelennamine, zolamine; piperazines such as cetirizine, chlorcyclizine, clocinizine, hydroxyzine; phenothiazines such as ahistan, etymemazine, fenethazine, N-hydroxyethylpromethazine, isopromethazine, mequitazine, promethazine, thiazinamium methyl sulfate; other tricyclics such as azatadine, clobenzepam, cyproheptadine, deptropine, isothipendyl, loratadine; others such as antazoline, astemizole, azelastine, cetoxime, clemizole, clobenztropine, ebastine, emedastine, epinastine, fexofenadine, levocabastine, mebhydroline, phenindamine, terfenadine, tritoqualine; or any pharmaceutically acceptable addition salts thereof.

The amount of antiallergic or antihistaminic is present in the ordinary dosage intended to elicit and to obtain the intended effect. Such dosages are known to the pharmaceutical skilled artisans or practitioner in the medical arts and are not part or claimed in the present invention. In a preferred embodiment the antiallergic or antihistaminic dosage to elicit the desired effect is in the range of from 0.5 % to 5.0 %, preferably of from 1.0 % to 3.0 % by weight, this depending mainly on the specific pharmacological activity of the therapeutically effective agent.

### M-Embodiment for Topical Immunosuppressor (TNF-alpha inhibitor) Treatments

In a further preferred embodiment a therapeutically effective agent that may be useful in treatment of topical inflammatory conditions, refractory to conventional therapy of corticosteroids, comprises a topical immunsuppressor (TNF-alpha inhibitor) such as tacrolimus, pimecrolimus, imiquimod. In fact these effective agents may be indicated for the treatment of moderate and serious atopic dermatitis, wherein extensive body surfaces may be treated, thus representing a remarkable advantage over the existing medications in the form of cream or ointment.

The preferred quantity of said substance useful in treatment of atopic dermatitis, refractory to the conventional corticosteroids, in the pharmaceutical composition of the invention is in the range of from 0.03 % to 1.0 %, preferably of about 0.1 % by weight, this depending on the pathology to be treated and on the duration of treatment.

A remarkable advantage of the invention is that the pharmaceutical pressurized fill composition is non occlusive, so that, when applied on the skin, is resulting highly breathable. Therefore the patient's compliance is improved considerably as the novel system does not occlude the skin or membrane and therefore the pharmaceutical composition of the invention is minimizing the local irritation and allergic sensitization problems arising from prolonged exposure of the skin to both the therapeutically active agent and the propellant mixture.

Another remarkable advantage of the pharmaceutical composition of the invention is that the pressurized propellant mixture is a pressurized liquid and therefore the therapeutically effective agent is more homogeneously solubilized, dispersed or emulsified inside the pressurized aerosol container. As a direct consequence of the above, the delivered sprayed mixture contains a homogeneous percentage of the therapeutically effective agent.

In another further advantage the pharmaceutical composition of the invention can be easily used to treat extensive and seriously affected areas, without any mechanical trauma, as in the case of burns or wounds, thus assuring also an uniform and atraumatic distribution of the pharmaceutical composition on the affected area.

Additionally, the pharmaceutical pressurized composition of the invention is physiologically compatible with the skin conditions (neither excessively acidic nor basic), so as neither to cause nor to exacerbate the status of inflammation or the irritation of the treated area.

An additional technical advantage of the pharmaceutical composition of the invention is the stability, due to two combined advantages. The pharmaceutical pressurized fill composition is water-free, so that there is a very scarce possibility that the moisture or the water may damage or hydrolyse the therapeutically effective agent. Moreover the presence of a suitable preserving agent like chlorocresol or chloroxylenol or a mixture thereof at convenient concentrations controls and avoids the proliferation of possible bacterial contaminations, which may lead to the inactivation of the therapeutically effective agent. Therefore, the pharmaceutical compositions of the invention are showing satisfactory stability patterns during the storage period comprised between the production and their use.

The pharmaceutical pressurized filling composition is preferably dispensed by spraying it from an aerosol dispenser. Therefore the pharmaceutical filling composition of the invention may be conveniently prepared by dividing the desired weight or volume into a suitable container (from glass or from metal, preferably from aluminium) which is sealed with a suitable pressure-valve system and filled with the propellant to reach the desired weight and convenient pressure (generally at about 3 bar pressure). The container volume of the pharmaceutical fill composition is typically ranging from 10 ml to 200 ml. Larger volumes are not advisable due to the presence of inflammable aliphatic hydrocarbons propellants.

The invention thus also relates to the pharmaceutical dosage form based on the pharmaceutical composition defined above. Moreover, the invention comprises a method of using the inventive pharmaceutical pressurized composition pursuant to which with an optional metered valve a metered dose of the medicinal fill is dispensed onto an intended application site. The intended application site is preferably skin, and the metered dose is preferable applied over a fixed surface area.

The invention will now be described with reference to the following examples.

### EXAMPLE 1

### Preparation of 1,000 cans of pharmaceutical aerosol composition (200.0 ml) for topical treatment of infected wounds.

The manufacturing steps were carried out at a bacteriologically controlled area, suitably equipped with an aerosol filling station under laminar air flow, being the pharmaceutical installations operated at the following environmental conditions of relative humidity (R.H.): 35 % - 50 % and of temperature (t°): 22° ± 3° C.

Quali-quantitative composition for the individual unit and for a batch of 1,000 cans:

| Ingredients | 200.0 ml can g / can | 1,000 cans Total Kg |
|---|---|---|
| Polymyxin B sulfate | 200,000 I. U. | --- |
| (6,000 I.U. = 1.0 mg) | (0.033) | 0.033 |
| Zinc bacitracin | 10,000 I.U. | --- |
| (10,000 = 166.0 mg) | (0.166) | 0.166 |
| Isopropyl myristate | 1.600 | 1.600 |
| Cholesterol | 0.180 | 0.180 |
| Chlorocresol | 0.002 | 0.002 |
| Propellant mixture 1 | 88.199 | 88.199 |
| Filling mixture | 9̅0̅.̅1̅8̅0̅ | 9̅0̅.̅1̅8̅0̅ |

The preparation of 1.981 Kg of the pharmaceutical fill composition was carried out according to the methods well known to skilled artisans experienced with topical aerosol preparations. After fractioning under laminar air flow 1.981 g of filling mixture into each of the 1,000 cans (200.0 ml volume), each can was closed by sealing it with the pressure valve round portion.

Thereafter the sealed can was filled with the gas propellant mixture 1 (88.199 g/can) under a convenient pressure through the stem valve until the desired weight of the can was obtained. Finally a dispensing nozzle reciprocating with the valve stem was installed.

962 units of 200.0 ml cans of pharmaceutical aerosol composition for topical use, suitable in the treatment of infected wounds were obtained. Final production yield: 96.20 %

### EXAMPLE 2

### Preparation of 1,200 cans of pharmaceutical aerosol composition (40.0 ml) suitable in analgesic topical treatments.

The manufacturing steps were carried out at pharmaceutical installations operated at the following environmental conditions of relative humidity (R.H.): 35 % - 50 % and of temperature (t°): 22°± 3° C.

Quali-quantitative composition for the individual unit and for a batch of 1,200 cans:

| Ingredients | 40.0 ml can g / can | 1,200 cans Total Kg |
|---|---|---|
| Etofenamate (*) | 2.000 | 2.400 |
| Isopropyl myristate | 3.800 | 4.560 |
| Isopropyl palmitate | 1.200 | 1.440 |
| Ethanol (anhydrous) | 2.800 | 3.360 |
| Cholesterol | 0.160 | 0.192 |
| Chlorocresol | 0.005 | 0.006 |
| Propellant mixture 4 | 24.300 | 29.160 |
| Filling mixture | 3̅4̅.̅2̅6̅5̅ | 4̅1̅.̅1̅1̅8̅ |

| | | |
|---|---|---|
| (*) as 100 % etofenamate | | |

The preparation of 11.958 Kg of the pharmaceutical fill composition was carried out according to the methods well known to skilled artisans experienced with topical aerosol preparations. After fractioning 9.965 g of filling mixture into each of the 1,200 cans (40.0 ml volume), each can was closed by sealing it with the pressure valve round portion.

Thereafter the sealed can was filled with the gas propellant mixture 4 (24.3 g/can) under a convenient pressure through the stem valve until the desired weight of the can was obtained. Finally a dispensing nozzle reciprocating with the valve stem was installed.

1,173 units of 40.0 ml cans of pharmaceutical aerosol composition, suitable in analgesic topical treatments were obtained. Final production yield: 97.75 %

### EXAMPLE 3

### Preparation of 2,000 cans of pharmaceutical aerosol composition (30.0 ml) for topical treatment of burns.

The manufacturing steps were carried out at a bacteriologically controlled area, suitably equipped with an aerosol filling station under laminar air flow, being the pharmaceutical installations operated at the following environmental conditions of relative humidity (R.H.): 35 % - 50 % and of temperature (t°): 22°± 3°C.

Quali-quantitative composition for the individual unit and for a batch of 2,000 cans:

| Ingredients | 30.0 ml can g / can | 2,000 cans Total Kg |
|---|---|---|
| Clostebol acetate (*) | 0.150 | 0.300 |
| Neomycin sulfate (**) | 0.150 | 0.300 |
| Isopropyl myristate | 2.685 | 5.370 |
| Chlorocresol | 0.003 | 0.006 |
| Propellant mixture 3 | 16.412 | 32.824 |
| Filling mixture | 1̅9̅.̅4̅0̅0̅ | 3̅8̅.̅8̅0̅0̅ |

| | | |
|---|---|---|
| (*) as 100 % of clostebol acetate (**) to be increased to 100 % of neomycin base | | |

The preparation of 5.976 Kg of the pharmaceutical fill composition was carried out according to the methods well known to skilled artisans experienced with topical aerosol preparations. After fractioning under laminar air flow 2.988 g of filling mixture into each of the 2,000 cans (30.0 ml volume), each can was closed by sealing it with the pressure valve round portion.

Thereafter the sealed can was filled with the gas propellant mixture 3 (16.412 g/can) under a convenient pressure through the stem valve until the desired weight of the can was obtained. Finally a dispensing nozzle applicator reciprocating with the valve stem was installed.

1,939 units of 30.0 ml cans of pharmaceutical aerosol composition for topical use, suitable in the treatment of burns were obtained. Final production yield: 96.95 %

### EXAMPLE 4

### Preparation of 1,500 cans of pharmaceutical aerosol composition (30.0 ml) suitable in antihistaminic topical treatments.

The manufacturing steps were carried out at pharmaceutical installations operated at the following environmental conditions of relative humidity (R.H.): 35 % - 50 % and of temperature (t°): 22° ± 3° C.

Quali-quantitative composition for the individual unit and for a batch of 1,500 cans:

| Ingredients | 30.0 ml can g / can | 1,500 cans Total Kg |
|---|---|---|
| Promethazine hydrochloride | 0.600 | 0.900 |
| Isopropyl myristate | 1.785 | 2.678 |
| Cholesterol | 0.125 | 0.187 |
| Ethanol (anhydrous) | 2.800 | 4.200 |
| Chlorocresol | 0.002 | 0.003 |
| Phenirat | 0.023 | 0.034 |
| Propellant mixture 4 | 16.500 | 24.750 |
| Filling mixture | 2̅1̅.̅8̅3̅5̅ | 3̅2̅.̅7̅5̅2̅ |

The preparation of 8.002 Kg of the pharmaceutical fill composition was carried out according to the methods well known to skilled artisans experienced with topical aerosol preparations. After fractioning 5.335 g of filling mixture into each of the 1,500 cans (30.0 ml volume), each can was closed by sealing it with the pressure valve round portion.

Thereafter the sealed can was filled with the gas propellant mixture 4 (16.5 g/can) under a convenient pressure through the stem valve until the desired weight of the can was obtained. Finally a dispensing nozzle reciprocating with the valve stem was installed.

1436 units of 30.0 ml cans of pharmaceutical aerosol composition, suitable in antihistaminic topical treatments were obtained. Final production yield: 95.73 %

### EXAMPLE 5

### Preparation of 1,800 glass bottles of pharmaceutical aerosol composition (30.0 ml) suitable in antiallergic-antihistaminic topical treatments.

The manufacturing steps were carried out at pharmaceutical installations operated at the following environmental conditions of relative humidity (R.H.): 35 % - 50 % and of temperature (t°): 22°± 3° C.

Quali-quantitative composition for the individual unit and for a batch of 1,800 glass bottles:

| Ingredients | 30.0 ml bottle g / bottle | 1.800 bottles Total Kg |
|---|---|---|
| Dexchlorpheniramine maleate | 0.300 | 0.540 |
| Isopropyl palmitate | 1.825 | 3.285 |
| cholesterol | 0.125 | 0.225 |
| Ethanol (anhydrous) | 2.550 | 4.590 |
| Chloroxylenol | 0.002 | 0.004 |
| Propellant mixture 4 | 16.800 | 30.240 |
| Filling mixture | 2̅1̅.̅6̅0̅2̅ | 3̅8̅.̅8̅8̅4̅ |

The preparation of 8.644 Kg of the pharmaceutical fill composition was carried out according to the methods well known to skilled artisans experienced with topical aerosol preparations. After fractioning under laminar air flow 4.802 g of filling mixture into each of the 1,800 glass bottles (30.0 ml volume), each bottle was closed by sealing it with the pressure valve.

Thereafter the sealed glass bottle was filled with the gas propellant mixture 4 (16.8 g/glass bottle) under a convenient pressure through the stem valve until the desired weight of the can was obtained. Finally a dispensing nozzle reciprocating with the valve stem was installed. 1704 units of 30.0 ml glass bottles of pharmaceutical aerosol composition, suitable in antiallergic-antihistaminic topical treatments were obtained.
Final production yield: 94.66 %

### EXAMPLE 6

### Preparation of 1, 600 cans of pharmaceutical aerosol composition (30.0 ml) suitable in antifungal topical treatments.

The manufacturing steps were carried out at pharmaceutical installations operated at the following environmental conditions of relative humidity (R.H.): 35 % - 50 % and of temperature (t°): 22° ± 3° C.

Quali-quantitative composition for the individual unit and for a batch of 1,600 cans:

| Ingredients | 30.0 ml can g / can | 1,600 cans Total Kg |
|---|---|---|
| Terbinafine hydrochloride | 0.300 | 0.480 |
| Isopropyl myristate | 2.390 | 3.824 |
| Cholesterol | 0.110 | 0.176 |
| Ethanol (anhydrous) | 4.200 | 6.720 |
| Chlorocresol | 0.004 | 0.006 |
| Propellant mixture 4 | 16.650 | 26.640 |
| Filling mixture | 2̅3̅.̅6̅5̅4̅ | 3̅7̅.̅8̅4̅6̅ |

The preparation of 11.206 Kg of the pharmaceutical fill composition was carried out according to the methods well known to skilled artisans experienced with topical aerosol preparations. After fractioning 7.004 g of filling mixture into each of the 1,600 cans (30.0 ml volume), each can was closed by sealing it with the pressure valve round portion.

Thereafter the sealed can was filled with the gas propellant mixture 4 (16.650 g/can) under a convenient pressure through the stem valve until the desired weight of the can was obtained. Finally a dispensing nozzle reciprocating with the valve stem was installed.

1,558 units of 30.0 ml cans of pharmaceutical aerosol composition, suitable in topical antifungal treatments were obtained.
Final production yield: 97.37 %

## Claims

1. A pharmaceutical composition comprising:
a) a substantially water-free pressurized propellant mixture comprising a homogeneous solution of:
at least 80.0% by weight of at least one pressure-liquefied propellant;
up to 15.0% by weight of at least one carrier;
from 0.001 % to 1.0% by weight of at least one chlorophenol derivative;
b) from 0.001 % to 5.0% by weight of at least one therapeutically effective agent or a variable mixture thereof.

2. A pharmaceutical composition according to claim 1, containing from 0.5% to 5.0% by weight of at least one emollient-humectant.

3. A pharmaceutical composition according to any one of claims 1 or 2, containing less than 5.0% by weight of at least one organic solvent.

4. A water-free pressurized propellant mixture according to claim 1, wherein the at least one pressure-liquefied propellant comprises a mixture of at least two aliphatic hydrocarbons, selected from n-butane, i-butane and propane.

5. A water-free pressurized propellant mixture according to any of claims 1 to 4 wherein the at least one carrier comprises isopropyl myristate or isopropyl palmitate or a variable mixture thereof.

6. A water-free pressurized propellant mixture according to any one of claims 1 to 5 wherein the chlorophenol derivative is purposely selected from chlorocresol and chloroxylenol or a variable mixture thereof.

7. A water-free pressurized propellant mixture according to any one of claims 1 to 6 wherein the at least one emollient-humectant is cholesterol.

8. A water-free pressurized propellant mixture according to any one of claims 1 to 7 wherein the at least one organic solvent is a low chain alcohol.

9. Use of chlorocresol or chloroxylenol or a variable mixture thereof in the water-free pressurized propellant mixture according to any one of claims 1 to 8 to control the microbiological count and to thereby avoid the sterilization by gamma-radiation of the aerosol dispenser at the end of the production process.

10. A pressurized pharmaceutical fill composition according to any one of claims 1 to 8 where at least one therapeutically effective agent or a variable mixture thereof admixed to the substantially water-free pressurized propellant mixture is comprised from 0.05% to 4.0% by weight.

11. A pressurized pharmaceutical fill composition according to claim 10 wherein the at least one therapeutically effective agent is effective in one or more of the treatment of burns, wounds, abrasions, delayed skin healing conditions, and also topical fungal infections, topical bacterial infections, topical viral infections, acne, seborrhea, keratitis, psoriasis, hair loss, topical painful conditions, topical inflammatory conditions, the promotion of anaesthetic effects, the provision of antiallergic, antihistaminic effects and also topical immunosuppressor treatment.

12. A pressurized pharmaceutical fill composition according to claims 10 and 11 wherein the at least one therapeutically effective agent comprises one or more healing enhancers and one or more antibacterial or antibiotic agents or a variable mixture thereof.

13. A pressurized pharmaceutical fill composition according to claim 12 comprising:
about 85.0% by weight of pressure liquefied propellant comprising n-butane, i-butane and propane in the volume ratio about 1:15:4;
about 14.0% by weight of isopropyl myristate;
about 0.015% by weight of chlorocresol;
about 0.08% by weight of clostebol acetate; and
about 0.08% by weight of neomycin sulfate.

14. An aerosol dispenser containing a pressurized pharmaceutical fill composition according to claim 10.

15. A method of filling an aerosol dispenser according to claim 14, comprising the steps of:
filling a suitable container with the desired weight or volume of the carrier, the bactericidal disinfectant, the therapeutically effective agent, and optionally the emollient-humectant and the organic solvent;
sealing the filled container with a pressure-valve system; and
further filling the container with a pressure-liquefied propellant.

## Patentansprüche

1. Pharmazeutische Zusammensetzung enthaltend :
a) nahezu wasserfreies komprimiertes Treibmittelgemisch als homogene Lösung, bestehend aus :
einem druckverflüssigten Treibmittel mit wenigstens 80,0 Masse - %;
einem Trägerstoff mindestens bis zu 15,0 Masse - % ;
mindestens einem Chlorphenolderivat von 0,001 Masse - % bis 1,0 Masse - %;
b) mindestens einem therapeutischen Wirkstoff von 0,001% bis 5,0% oder einer variablen Mischung solcher Wirkstoffe.

2. Pharmazeutische Zusammensetzung nach Anspruch 1, die mindestens von 0,5 Masse - % bis 5,0 Masse - % ein weichmachendes Feuchtigkeitsmittel enthält.

3. Pharmazeutische Zusammensetzung nach Anspruch 1 oder 2, die zumindest organisches Lösungsmittel von weniger als 5,0 Masse - % enthält.

4. Wasserfreies komprimiertes Treibmittelmischung nach Anspruch 1, **dadurch gekennzeichnet, dass** zumindest ein druckverflüssigtes Treibmittel eine Mischung aus wenigstens zwei aliphatischen Kohlenwasserstoffen enthält, die aus den Verbindungen n-Butan, i-Butan und Propan ausgewählt sind.

5. Wasserfreies komprimiertes Treibmittelgemisch nach einem oder mehreren der Ansprüche 1 bis 4, **dadurch gekennzeichnet, dass** zumindest ein Trägerstoff die Verbindung Isopropylmyristat oder Isopropyl Palmitate oder eine variable Mischung aus beiden enthält.

6. Wasserfreies komprimiertes Treibmittelgemisch nach einem oder mehreren der Ansprüche 1 bis 5 , **dadurch gekennzeichnet, dass** als Chlorophenolderivat entweder Chlorocresol oder Chlorxylenol oder eine Mischung aus beiden eingesetzt wird.

7. Wasserfreies komprimiertes Treibmittelgemisch nach einem oder mehreren der Ansprüche 1 bis 6, **dadurch gekennzeichnet, dass** zumindest ein weichmachendes Feuchtigkeitsmittel Cholesterol ist.

8. Wasserfreies komprimiertes Treibmittelgemisch nach einem oder mehreren der Ansprüche 1 bis 7, **dadurch gekennzeichnet, dass** zumindest ein organisches Lösungsmittel ein kurzkettiger Alkohol ist.

9. Verwendung von Chlorocresol oder Chlorxylenol oder einer variablen Mischung aus beiden in dem wasserfreien komprimierten Treibmittelgemisch nach einem oder mehreren der Ansprüche 1 bis 8 , um die mikrobiologische Belastung (Bioburden) zu kontrollieren und somit die Sterilisierung durch Gammastrahlen der Aerosolpackung am Ende des Produktionsprozesses zu vermeiden.

10. Komprimiertes pharmazeutisches Füllprodukt nach einem oder mehreren der Ansprüche 1 bis 8, wobei zumindest ein therapeutischer Wirkstoff oder eine variable Mischung aus mehreren Wirkstoffen dem wasserfreien komprimierten Treibmittelgemisch mit einem Gewichtsanteil von 0,05% bis 4,0% beigegeben ist.

11. Komprimiertes pharmazeutisches Füllprodukt nach Anspruch 10, worin zumindest ein therapeutischer Wirkstoff erfolgreich eingesetzt wird bei der Behandlung oder den Behandlungen von Brandwunden, Wunden, Abschürfungen, verschleppter Wundheilung bei Hautverletzungen, sowie lokal begrenzten Pilzinfektionen, lokal begrenzten bakteriellen Infektionen, lokal begrenzten viralen Infektionen, Akne, Seborrhea, Keratitis, Psoriasis, Haarausfall, lokal begrenzten Schmerzen, viralen Infektionen, Entzündungen, zur Förderung der Schmerzlinderung, zur Erzielung antiallergischer, antihistaminischer Wirkungen als auch bei der Behandlung mit Immunsuppressiva.

12. Komprimiertes pharmazeutisches Füllprodukt nach Anspruch 10 und 11, **dadurch gekennzeichnet, dass** zumindest ein therapeutischer Wirkstoff einen oder mehrere Heilungsverstärker oder einen oder mehrere antibakterielle oder antibiotische Wirkstoffe oder eine Mischung solcher Stoffe enthält.

13. Komprimiertes pharmazeutisches Füllprodukt nach Anspruch 12, **dadurch gekennzeichnet, dass** es ungefähr:
85,0 Masse - % druckverflüssigtes Treibmittel mit n-Butan, i-Butan und Propan im Massenverhältnis 1:15:4;
14,0 Masse - % Isopropylmyristat;
0,015 Masse - % Chlorocresol;
0,08 Masse - % Clostebol Acetat; und
0,08 Masse - % Neomycin Sulfat
enthält.

14. Aerosolpackung mit dem komprimierten pharmazeutischen Füllprodukt nach Anspruch 10.

15. Befüllungsmethode für Aerosolpackung nach Anspruch 14, **dadurch gekennzeichnet, dass** sie aus folgenden Schritten besteht:
Befüllung eines passenden Behälters mit dem gewünschten Gewicht oder
Volumen des Trägerstoffes, des keimtötenden Desinfektionsmittels, des therapeutischen Wirkstoffes und optional des pflegenden Feuchtigkeitsstoffes und des organischen Lösungsmittels;
Versiegelung des befüllten Behälters mit einem Sprühkopf- and
Ventilsysteme; Befüllung des Behälters mit einem druckverflüssigten Treibmittel.

## Revendications

1. Une composition pharmaceutique comprenant :
a) un mélange propulseur sous pression sensiblement exempt d'eau comprenant une solution homogène de :
au moins 80,0 % en poids d'au moins un propulseur liquéfié sous pression ;
jusqu'à 15,0 % en poids d'au moins un support ;
de 0,001 % à 1,0 % en poids d'au moins un dérivé de chlorophénol.
b) de 0,001 % à 5,0 % en poids d'au moins un agent thérapeutiquement efficace ou d'un mélange variable de celui-ci.

2. Une composition pharmaceutique selon la revendication 1 contenant de 0,5 % à 5,0 % en poids d'au moins un émollient-humectant.

3. Une composition pharmaceutique selon la revendication 1 ou 2 contenant moins de 5,0 % en poids d'au moins un solvant organique.

4. Un mélange propulseur sous pression exempt d'eau selon la revendication 1 dans lequel au moins un propulseur liquéfié sous pression comprend un mélange d'au moins deux hydrocarbures aliphatiques sélectionnés à partir de n-butane, de i-butane et de propane.

5. Un mélange propulseur sous pression exempt d'eau selon une des revendications de 1 à 4 dans lequel au moins un support comprend du myristate d'isopropyle ou du palmitate d'isopropyle ou un mélange variable de ceux-ci.

6. Un mélange propulseur sous pression exempt d'eau selon une des revendications de 1 à 5 dans lequel le dérivé de chlorophénol est sélectionné intentionnellement à partir du chlorocrésol et du chloroxylènol ou d'un mélange variable de ceux-ci.

7. Un mélange propulseur sous pression exempt d'eau selon une des revendications de 1 à 6 dans lequel au moins un émollient-humectant est du cholestérol.

8. Un mélange propulseur sous pression exempt d'eau selon une des revendications de 1 à 7 dans lequel au moins un solvant organique est un alcool à chaîne courte.

9. Utilisation de chlorocrésol ou de chloroxylènol ou d'un mélange variable de ceux-ci dans le mélange propulseur sous pression exempt d'eau selon une des revendications de 1 à 8 pour contrôler la numération microbiologique et ainsi éviter la stérilisation par rayonnement gamma du générateur d'aérosol à la fin du processus de production.

10. Une composition pharmaceutique de remplissage sous pression selon une des revendications de 1 à 8 dans laquelle au moins un agent thérapeutiquement efficace ou un mélange variable de ce dernier incorporé au mélange propulseur sous pression sensiblement exempt d'eau est compris entre 0,05 % et 4,0 % en poids.

11. Une composition pharmaceutique de remplissage sous pression selon la revendication 10 dans laquelle au moins un agent thérapeutiquement efficace est efficace dans un ou plusieurs des traitements des brûlures, des plaies, des abrasions, des affections de cicatrisation retardée, et également des infections fongiques localisées, des infections bactériennes localisées, des infections virales localisées, de l'acné, de la séborrhée, de la kératite, du psoriasis, de la chute de cheveux, des affections douloureuses localisées, des affections inflammatoires localisées, la promotion d'effets anesthésiants, obtention d'effets antiallergiques, d'effets antihistaminiques et également d'un traitement immunosuppresseur topique.

12. Une composition pharmaceutique de remplissage sous pression selon les revendications 10 et 11 dans laquelle au moins un agent thérapeutiquement efficace comprend un ou plusieurs agents antibactériens ou antibiotiques ou un mélange variable de ceux-ci.

13. Une composition pharmaceutique de remplissage sous pression selon la revendication 12 comprenant :
environ 85,0 % en poids de propulseur liquéfié sous pression comprenant du n-butane, du i-butane et du propane dans un rapport de volume d'environ 1:15:4 ;
environ 14,0 % en poids de myristate d'isopropyle ;
environ 0,015 % en poids de chlorocrésol ;
environ 0,08 % en poids d'acétate de clostébol ; et
environ 0,08 % en poids de sulfate de néomycine.

14. Un générateur d'aérosol contenant une composition pharmaceutique de remplissage sous pression selon la revendication 10.

15. Une méthode de remplissage d'un générateur d'aérosol selon la revendication 14 comprenant les étapes suivantes :
remplissage du conteneur adapté avec le poids ou le volume souhaité de support, le désinfectant bactéricide, l'agent thérapeutiquement efficace, et éventuellement l'émollient-humectant et le solvant organique ;
scellage du conteneur rempli avec un système de valve de pression ; et
remplissage supplémentaire du conteneur avec un propulseur liquéfié sous pression.
